(19) Europäisches Patentamt
European Patent Office
Office européen des brevets

(11) **EP 2 749 209 A1**

(12) **EUROPEAN PATENT APPLICATION**

(43) Date of publication:
**02.07.2014 Bulletin 2014/27**

(51) Int Cl.:
**A61B 5/00** *(2006.01)* **G01N 29/24** *(2006.01)*
**G01N 21/17** *(2006.01)*

(21) Application number: **13197073.3**

(22) Date of filing: **13.12.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.12.2012 JP 2012286684**

(71) Applicant: **Canon Kabushiki Kaisha Tokyo 146-8501 (JP)**

(72) Inventors:
• **Watanabe, Tadaki Tokyo, Tokyo 146-8501 (JP)**
• **Abe, Hiroshi Tokyo, Tokyo 146-8501 (JP)**

(74) Representative: **TBK Bavariaring 4-6 80336 München (DE)**

(54) **Object information acquisition apparatus, display method, and program**

(57)    An object information acquisition apparatus disclosed in the present specification includes a light source (110), acoustic wave detection means (130), and processing means (140). The light source (110) is configured to emit light. The acoustic wave detection means (130) is configured to detect a photoacoustic wave generated by irradiation of an object with the light and to output a detection signal. The processing means (140) is configured to be able to acquire two or more types of object information on the basis of the detection signal and configured to cause display means (160) to display at least one type of object information selected by a user from among the two or more types of object information.

FIG. 3

EP 2 749 209 A1

**Description**

BACKGROUND OF THE INVENTION

Field of the Invention

The present invention relates to a technology used to obtain information on an object on the basis of a photoacoustic wave generated by irradiation of the object with light.

Description of the Related Art

Photoacoustic imaging (PAI) using a photoacoustic effect is one example of optical imaging technologies using light. In photoacoustic imaging, for example, an object such as a living body is irradiated with pulsed light and a photoacoustic wave is generated by a light absorber such as a blood vessel absorbing the energy of the pulsed light. A photoacoustic wave generated by this photoacoustic effect is detected by an acoustic wave detection unit. Then, object information is acquired by performing analysis processing on a detection signal output from the acoustic wave detection unit.

Here, an initial sound pressure P of a photoacoustic wave generated by the photoacoustic effect is expressed by the following equation 1. $\Gamma$ denotes a Grüneisen coefficient, and is obtained by dividing the product of a coefficient of volume expansion $\beta$ and the square of the speed of sound c by a specific heat at a constant pressure $C_P$. When an object is set, $\Gamma$ has an almost constant value corresponding to the object. $\mu_a$ denotes an absorption coefficient of a region of interest, and $\Phi$ denotes the light intensity of a region of interest.

$$P = \Gamma \cdot \mu_a \cdot \Phi \qquad (\text{Eq. 1})$$

Japanese Patent Laid-Open No. 2010-088627 discloses acquisition of an initial sound pressure distribution of the inside of a living body on the basis of a photoacoustic wave generated by a photoacoustic effect. Furthermore, using Equation 1, Japanese Patent Laid-Open No. 2010-088627 discloses acquisition of an absorption coefficient distribution of the inside of a living body by dividing an initial sound pressure distribution of the inside of the living body by a light intensity distribution of light that has propagated in the living body, the absorption coefficient distribution of the inside of the living body serving as object information.

SUMMARY OF THE INVENTION

The present invention in its first aspect provides an object information acquisition apparatus as specified in claims 1 to 11.
The present invention in its second aspect provides a display method as specified in claims 12 to 14.
The present invention in its third aspect provides a program as specified in claim 15.
Further features of the present invention will become apparent from the following description of exemplary embodiments with reference to the attached drawings.

BRIEF DESCRIPTION OF THE DRAWINGS

Fig. 1 is a diagram illustrating an object information acquisition apparatus according to an exemplary embodiment and an example.
Fig. 2 is a diagram illustrating details of a processing unit according to the exemplary embodiment and the example.
Fig. 3 is a diagram illustrating the flow of an object information acquisition method according to the exemplary embodiment.
Fig. 4 is a diagram illustrating the flow of an object information acquisition method according to the example.
Fig. 5 is a diagram illustrating an initial sound pressure distribution corresponding to 756-nm light according to the example.
Fig. 6A is a diagram illustrating an oxygen saturation distribution according to the example.
Fig. 6B is a diagram illustrating display of another oxygen saturation distribution according to the example.

DESCRIPTION OF THE EMBODIMENTS

In Japanese Patent Laid-Open No. 2010-088627, an absorption coefficient distribution of the inside of an object is acquired using light of a certain wavelength at which hemoglobin absorbs more light. This absorption coefficient distribution is displayed on a display unit.

An absorption coefficient distribution acquired using a method such as one method described in Japanese Patent Laid-Open No. 2010-088627 is a distribution which shows light absorption performed mainly by blood vessels. Thus, such an absorption coefficient distribution is effective for determining the shapes and positions of blood vessels. Malignant tumors consume a lot of oxygen, and thus many blood vessels are formed around malignant tumors. Display of an absorption coefficient distribution helps users to determine the shapes and positions of blood vessels and is consequently useful for diagnosis of the presence of a malignant tumor.

In this manner, in photoacoustic imaging, morphological information such as an absorption coefficient distribution may be acquired from a detection signal of a photoacoustic wave generated by performing irradiation with light of a single wavelength, the morphological information enabling the shape of an object to be determined. Then, a user may make a diagnosis of the presence of a malignant tumor by checking displayed morphological information.

However, even though the shape of an object may be determined using such morphological information, it is difficult to quantitatively evaluate the state of the object. Thus, in diagnosis using morphological information, the accuracy of diagnosis may be decreased.

In contrast, in photoacoustic imaging, spectral information may be acquired from detection signals of photoacoustic waves generated by performing irradiation with light of a plurality of wavelengths, the spectral information enabling the density of a substance in the object and the like to be determined. Here, each of the photoacoustic waves is generated by performing irradiation with light of a corresponding one of the plurality of wavelengths.

In the following, an oxygen saturation distribution will be described as an example of spectral information. Since malignant tumors consume a lot of oxygen, oxygen saturation around a malignant tumor is lower than that around normal tissue. Thus, a user may make a diagnosis of the presence of a malignant tumor by checking a displayed oxygen saturation distribution.

However, since spectral information such as oxygen saturation is obtained using the ratio between absorption coefficients of light of a plurality of wavelengths, spectral information is displayed due to the ratio between random noises or the like even in a region outside an observation target such as blood vessels. Here, each of the absorption coefficients corresponds to light of a corresponding one of the plurality of wavelengths. Since spectral information is displayed for both an observation target and a region other than the observation target, it is difficult to distinguish one from another. As a result, when a diagnosis is made using spectral information, the accuracy of diagnosis may be decreased.

In the following, a method for acquiring oxygen saturation, which is an example of spectral information acquired using light of two wavelengths, will be described.

For example, a molar absorption coefficient of oxyhemoglobin is denoted by $\varepsilon_{HbO}$ ($mm^{-1}M^{-1}$) and a molar absorption coefficient of deoxyhemoglobin is denoted by $\varepsilon_{Hb}$ ($mm^{-1}M^{-1}$). Here, a molar absorption coefficient refers to an absorption coefficient obtained when there is 1 mol of hemoglobin per liter. In addition, a molar absorption coefficient is uniquely determined by wavelength.

In addition, the density (M) of oxyhemoglobin is denoted by $C_{HbO}$ and the density (M) of deoxyhemoglobin is denoted by $C_{Hb}$. Here, absorption coefficients $\mu_a$ acquired by using light of a first wavelength $\lambda_1$ and light of a second wavelength $\lambda_2$ may be expressed as Equation (2).

$$\begin{cases} \mu_a(\lambda_1) = \varepsilon_{HbO}(\lambda_1) \cdot C_{HbO} + \varepsilon_{Hb}(\lambda_1) \cdot C_{Hb} \\ \mu_a(\lambda_2) = \varepsilon_{HbO}(\lambda_2) \cdot C_{HbO} + \varepsilon_{Hb}(\lambda_2) \cdot C_{Hb} \end{cases} \quad (Eq.\ 2)$$

That is, for each wavelength, the absorption coefficient $\mu_a$ of the wavelength is expressed as the sum of the product of the molar absorption coefficient of oxyhemoglobin and the density of oxyhemoglobin and the product of the molar absorption coefficient of deoxyhemoglobin and the density of deoxyhemoglobin.

In addition, the density of oxyhemoglobin and the density of deoxyhemoglobin may be expressed as Equation (3), which is derived from Equation (2).

$$\begin{cases} C_{HbO} = \dfrac{\varepsilon_{Hb}(\lambda_1)\cdot\mu_a(\lambda_2) - \varepsilon_{Hb}(\lambda_2)\cdot\mu_a(\lambda_1)}{\varepsilon_{Hb}(\lambda_1)\cdot\varepsilon_{HbO}(\lambda_2) - \varepsilon_{HbO}(\lambda_1)\cdot\varepsilon_{Hb}(\lambda_2)} \\[2em] C_{Hb} = \dfrac{\varepsilon_{Hb}(\lambda_1)\cdot\mu_a(\lambda_2) - \varepsilon_{HbO}(\lambda_2)\cdot\mu_a(\lambda_1)}{\varepsilon_{HbO}(\lambda_1)\cdot\varepsilon_{Hb}(\lambda_2) - \varepsilon_{Hb}(\lambda_1)\cdot\varepsilon_{Hb}(\lambda_2)} \end{cases} \qquad (\text{Eq. } 3)$$

Oxygen saturation $StO_2$ represents a proportion of oxyhemoglobin in all types of hemoglobin and may be expressed as Equation (4) shown below.

$$StO_2 = \frac{C_{HbO}}{C_{HbO} + C_{Hb}} = \frac{-\mu_a(\lambda_1)\varepsilon_{Hb}(\lambda_2) + \mu_a(\lambda_2)\varepsilon_{Hb}(\lambda_1)}{-\mu_a(\lambda_1)\{\varepsilon_{Hb}(\lambda_2) - \varepsilon_{HbO}(\lambda_2)\} + \mu_a(\lambda_2)\{\varepsilon_{Hb}(\lambda_1) - \varepsilon_{HbO}(\lambda_1)\}}$$

$(\text{Eq. } 4)$

As expressed by Equation (4), the oxygen saturation $StO_2$ is obtained from the ratio between the absorption coefficient $\mu_a$ for the first wavelength ($\lambda_1$) and the absorption coefficient $\mu_a$ for the second wavelength ($\lambda_2$). Thus, as described above, for spectral information such as the oxygen saturation $StO_2$, it is difficult to distinguish an observation target from a region other than the observation target.

In this manner, a pathological value of object information obtained in photoacoustic imaging varies depending on the type of the object information. In addition, causes of a decrease in the accuracy of diagnosis vary depending on the type of object information obtained in the photoacoustic imaging.

Thus, the inventors conceived the idea of causing a display unit to display a desired type of object information selected by a user from among two or more types of object information acquired on the basis of a detection signal of a photoacoustic wave. As a result, the inventors found that a user may make a diagnosis using a certain type of object information having a pathological value desired by the user among two or more types of object information having different pathological values. In addition, for a certain type of object information, a cause of a decrease in the accuracy of diagnosis may be compensated by using a different type of object information.

In particular, morphological information and spectral information are information that tend to readily complement each other's characteristics. Morphological information is suitable for determining the shapes and positions of blood vessels and the like, which are observation targets, but is not suitable for quantitative evaluation of the state of an observation target. In contrast, spectral information is not suitable for determining the shapes and positions of observation targets, but is suitable for quantitative evaluation of the state of an observation target. Thus, it is possible for morphological information and spectral information to complement each other by comparing the morphological information and the spectral information.

Examples of morphological information obtained using light of a single wavelength include an initial sound pressure distribution of a photoacoustic wave generated by a photoacoustic effect; and an optical energy absorption density distribution, an absorption coefficient distribution, and the like derived from the initial sound pressure distribution.

Examples of spectral information obtained using light of a plurality of wavelengths include the density and the like of substances that constitute tissue. Here, examples of the density of a substance include an oxygen saturation distribution, an oxyhemoglobin density distribution, a deoxyhemoglobin density distribution, and the like.

Alternatively, information obtained by performing correction on a certain type of object information may be used as object information according to this exemplary embodiment, the correction being, for example, modification using a weight based on a different type of object information. For example, the product of an absorption coefficient distribution and an oxygen saturation distribution may be used as object information according to this exemplary embodiment.

In the following, with reference to the drawings, this exemplary embodiment of the present invention will be described in detail. Note that, as a rule, the same structural elements are denoted by the same reference numerals and description thereof will be omitted.

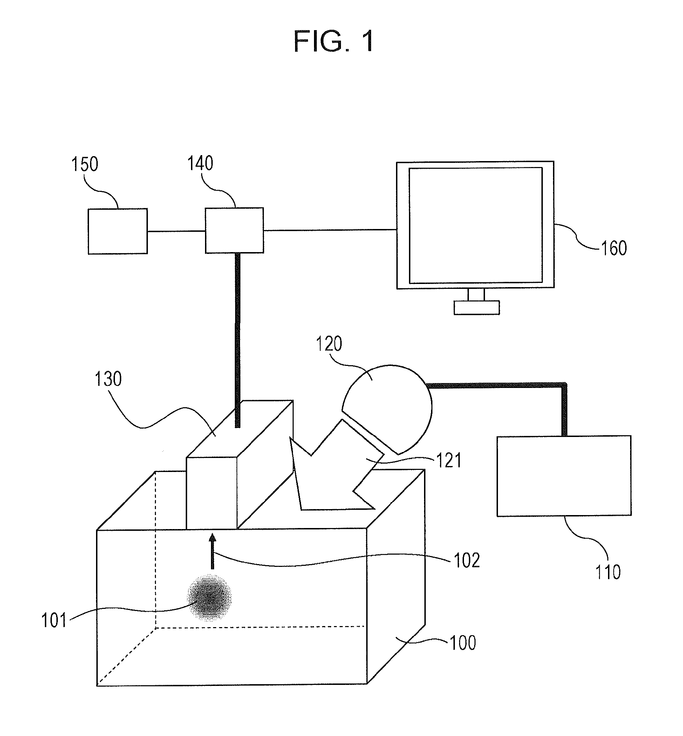

First, a basic structure of an object information acquisition apparatus according to this exemplary embodiment will be described with reference to Fig. 1.

The object information acquisition apparatus according to this exemplary embodiment includes, to acquire information on a living body 100 serving as an object, a light source 110, an optical system 120, an acoustic wave detection unit 130, a processing unit 140 serving as a computer, an input unit 150, and a display unit 160.

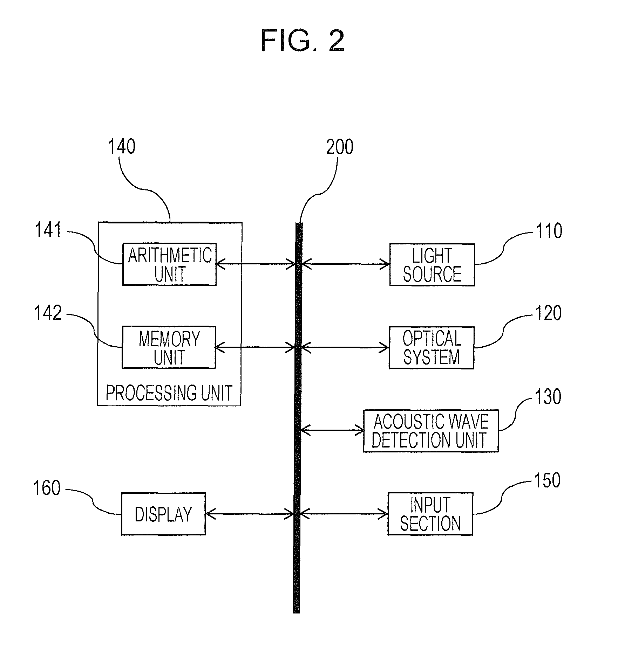

Fig. 2 is a schematic diagram illustrating connection with each element included in the object information acquisition apparatus according to this exemplary embodiment. As illustrated in Fig. 2, the processing unit 140 includes an arithmetic section 141 and a memory section 142.

The arithmetic section 141 controls, via a bus 200, operation of units that constitute the object information acquisition apparatus. In addition, the arithmetic section 141 reads a program that is stored in the memory section 142 and in which an object information acquisition method described below is described, and causes the object information acquisition apparatus to execute the object information acquisition method.

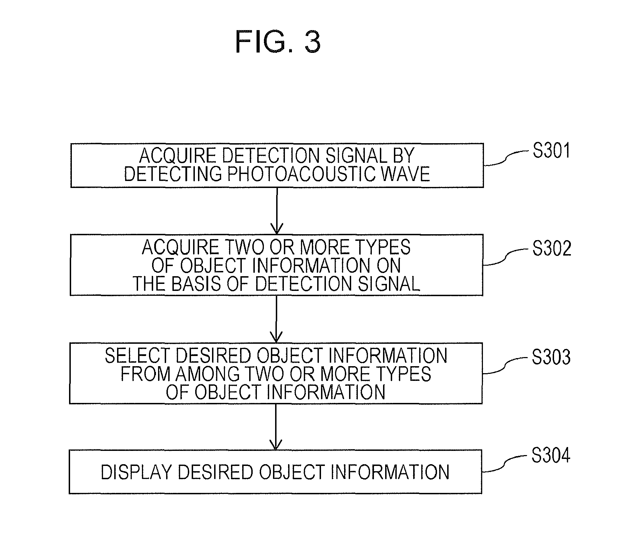

Next, an object information acquisition method according to this exemplary embodiment using the object information acquisition apparatus illustrated in Fig. 1 will be described with reference to a flow chart illustrated in Fig. 3. S301: Process for Acquiring Detection Signal by Detecting Photoacoustic Wave

In this process, first, the living body 100 is irradiated with light emitted by the light source 110 as pulsed light 121 via the optical system 120. Then, the pulsed light 121 used for irradiation is absorbed by a light absorber 101, and a photoacoustic wave 102 is generated by a photoacoustic effect.

Then, the acoustic wave detection unit 130 detects the photoacoustic wave 102 and outputs a detection signal to the processing unit 140. The detection signal output from the acoustic wave detection unit 130 is stored in the memory section 142 as detection signal data.

Note that, signals that are output from the acoustic wave detection unit 130 and have not yet converted into object information are collectively called detection signals in this exemplary embodiment of the present invention. That is, detection signals that are analog signals output from the acoustic wave detection unit 130, digital signals obtained by performing A/D conversion on analog signals, signals obtained by amplifying these analog signals or digital signals, and the like are detection signals in this exemplary embodiment of the present invention. In addition, even in the case where signals output from the acoustic wave detection unit 130 are added to each other, when a signal obtained by addition is not object information that has a pathological value, the signal obtained by addition is also a detection signal in this exemplary embodiment of the present invention.

S302: Process for Acquiring Two or More Types of Object Information on the basis of Detection Signal

In this process, the arithmetic section 141 acquires two or more types of object information on the basis of a detection signal stored in the memory section 142. Then, the arithmetic section 141 stores the two or more types of object information in the memory section 142.

Note that the arithmetic section 141 reads a program that is stored in the memory section 142 and in which an image reconstruction algorithm is described, and performs processing on detection signal data, the processing being based on the image reconstruction algorithm. Consequently, the arithmetic section 141 may acquire object information. As the image reconstruction algorithm, for example, a time-domain or Fourier-domain back projection method generally used in a tomography technology or Delay and Sum (D&S) may be used. Note that, in the case where a lot of time may be spent for reconstruction, an image reconstruction method such as an inverse analysis method using repetition processing may be used, too.

In addition, in photoacoustic imaging, optical characteristic information on the inside of a living body may be formed without performing image reconstruction by using a probe that performs reception focus using an acoustic lens or the like. In such a case, the processing unit 140 does not need to perform signal processing using an image reconstruction algorithm.

Note that, for each type of object information, the progress of calculation of the type of object information is preferably visualized on the display unit 160 by displaying a progress bar, an estimated calculation end time, or the like.

Note that, regardless of whether a certain type of object information has been selected or not in step S303 described below, a predetermined type of object information among two or more types of object information stored in the memory section 142 may be displayed on the display unit 160 in this process. Here, after performing imaging processing such as luminance value conversion or the like on the predetermined type of object information, which is read from the memory section 142, the arithmetic section 141 displays the resulting object information on the display unit 160. For example, setting is preferably set in advance such that an initial sound pressure for which calculation is completed faster than for the other types of object information, an absorption coefficient that may be acquired even with a single wavelength, or the like is displayed. Note that any type of object information may be displayed to meet the needs of a user that makes a diagnosis regardless of whether the type of object information is selected or not.

S303: Process for Selecting Desired Object Information from Two or More Types of Object Information

In this process, first, a user uses the input unit 150 and selects at least one type of object information from the two or more types of object information. Here, input information on desired object information is input from the input unit 150 and received by the processing unit 140.

In the following, a method for inputting object information desired by a user will be described. That is, the input unit 150 for selecting desired object information from among two or more types of object information will be described as an example. Note that as long as desired object information may be selected from among two or more types of object information, any method other than the method described below may be used.

For example, a user may select a desired type of object information by pressing a corresponding one of buttons serving as the input unit 150, each of the buttons corresponding to a corresponding one of the two or more types of object information. Alternatively, a desired type of object information may be selected by turning a corresponding one of dials serving as the input unit 150, each of the dials corresponding to a corresponding one of the two or more types of object information.

In addition, a user may select a desired type of object information by using a mouse, a keyboard, or the like serving as the input unit 150 and selecting an item displayed on the display unit 160 and representing the desired type of object information. Here, the display unit 160 may display items representing certain types of object information as icons so that they do not overlap with each other or may display as a menu. Moreover, items representing certain types of object information may be always displayed on the side of an image of object information on the display unit 160. Alternatively, such items may be displayed when a user has performed some kind of operation using the input unit 150. For example, the items representing certain types of object information may be displayed on the display unit 160 by clicking a button equipped with a mouse serving as the input unit 150.

Note that the desired object information is preferably at least one or more types of object information among the two or more types of object information. In addition, in this exemplary embodiment, at least two or more types of object information may be selected from among three or more types of object information. Here, the object information acquisition apparatus according to this exemplary embodiment may also be configured to be able to select a plurality of combinations of at least two types of object information. As a result, a type or types of object information desired by a user may be selected with a high degree of freedom, and a combination of certain types of object information effective for making a diagnosis may be displayed.

S304: Process for Displaying Desired Object Information

In this process, the arithmetic section 141 reads desired object information from among the two or more types of object information stored in the memory section 142 on the basis of input information input from the input unit 150 to the processing unit 140 in step S303. Subsequently, the arithmetic section 141 performs imaging processing such as luminance value conversion on the desired object information, outputs the resulting desired object information to the display unit 160, and causes the display unit 160 to display the resulting desired object information.

Note that the display unit 160 may display a plurality of types of object information in a superimposition manner or in a manner in which the plurality of types of object information do not overlap with each other.

In addition, display may be performed on the display unit 160 such that object information that has been displayed in advance is switched to object information that is newly selected. That is, the object information that has been displayed in advance (hereinafter referred to as previous object information) is hidden from view, and the object information that is newly selected is displayed in a region where the previous object information was displayed.

Note that a display method may be set in advance before shipment or a user may set a display method using the input unit 150.

In addition, preferably, the arithmetic section 141 performs luminance value conversion on the desired object information on the basis of a gray scale displayed in a color bar and causes the display unit 160 to display the resulting object information. Moreover, the unit of displayed object information may be displayed together with a color bar. For example, when oxygen saturation is displayed as object information, % may be displayed as the unit of oxygen saturation together with a color bar. Moreover, preferably, the input unit 150 is configured to allow a user to set a color to be displayed and assigned to a luminance value indicated by a color bar.

In addition, preferably, the input unit 150 is configured to allow a user to adjust a dynamic range of object information to be displayed. For example, as the input unit 150, which is used to adjust a dynamic range, a slide bar displayed on the display unit 160 or a mechanical system such as a dial may be used.

Moreover, a dynamic range that is adjustable may be changed in accordance with displayed object information.

As described in the above-described object information acquisition method according to this exemplary embodiment, since a user may select a type or types of object information to be displayed on a display, the user may make a diagnosis by collectively taking pieces of information having different pathological values into account.

In this exemplary embodiment, a display method has been described in which all of the two or more types of object information are acquired and stored in the memory section 142 in step S302 and thereafter the object information selected in step S303 is displayed in step S304. Note that, in this exemplary embodiment, a display method is not limited this as long as a certain type or types of object information selected from among two or more types of object information are displayed.

For example, when a user selects, using the input unit 150, an item representing a desired type of object information from among two or more types of object information, the arithmetic section 141 may start acquisition of the desired type of object information, which is selected, on the basis of a detection signal. Then, the arithmetic section 141 may cause the display unit 160 to display the desired type of object information acquired in this manner. In this case, too, the progress of calculation of each type of object information is preferably visualized on the display unit 160 by displaying a progress bar, an estimated calculation end time, or the like.

Note that, preferably, the types of object information that a user may select change in accordance with types of acquired object information. For example, the input unit 150 is preferably configured to be incapable of selecting a type of object information that has not yet been acquired on the basis of a detection signal. In addition, the input unit 150 is preferably configured to be incapable of selecting a type of object information that may be acquired only on the basis of a plurality of detection signals obtained using light of a plurality of wavelengths.

In addition, the object information acquisition apparatus according to this exemplary embodiment preferably includes a notification unit that notifies a user as to whether a subject type of object information is a type of object information that the user may select. Furthermore, this notification unit is preferably configured such that the user may visually recognize a type of object information that the user may select. For example, in the case where the display unit 160 displays items representing certain types of object information, items representing certain types of object information that a user may select may be displayed with a white background and items representing the other types of object information that a user is not allowed to select may be displayed with a gray background, or the like. In this manner, a user may be notified of certain types of object information that the user may select. In addition, a device included in the object information acquisition apparatus or a device outside the object information acquisition apparatus may be equipped with lamps serving as the notification unit, each of the lamps corresponding to a corresponding one of certain types of object information. In this case, the lamps corresponding to certain types of object information that the user may select are switched on and, for each type of object information, the user may be notified as to whether the type of object information may be selected or not.

Note that certain types of object information that a user is not allowed to select include, as described above, for example, a type of object information that has not yet been acquired by the processing unit 140 on the basis of a detection signal.

In addition, in the object information acquisition apparatus according to this exemplary embodiment, different types of object information corresponding to a certain time may be acquired from detection signals including a detection signal obtained by detecting an acoustic wave at the certain time. Two or more types of object information corresponding to the certain time are pieces of object information temporally shifted by a small amount from each other and thus are pieces of object information having different pathological values of a certain object that is in almost the same state.

In the object information acquisition apparatus according to this exemplary embodiment, desired object information selected by a user from among two or more types of object information acquired at the same time may be displayed. As a result, a diagnosis may be made by collectively taking, into account, pieces of information having different pathological values of a certain object that is in almost the same state.

Note that, in the object information acquisition apparatus according to this exemplary embodiment, it is not necessary to use a detection signal acquired at the same time for acquisition of the different types of object information.

In the following, details of the structure of the object information acquisition apparatus according to this exemplary embodiment will be described.

Light Source 110

The light source 110 is preferably a pulsed light source capable of generating pulsed light of the order of a few nanoseconds to a few microseconds. Specifically, in order to efficiently generate a photoacoustic wave, the light source 110 is preferably capable of generating light having a pulse width of the order of 10 nanoseconds. The wavelength of light that the light source 110 may emit is preferably the wavelength of light that propagates into the inside of an object. Specifically, in the case where an object is a living body, the wavelength of light is preferably from 500 nm to 1200 nm. In addition, in the case where a total hemoglobin density distribution is acquired using light of a single wavelength, the light source 110 that is capable of generating light of a wavelength at which an absorption coefficient of oxyhemoglobin is almost the same as an absorption coefficient of deoxyhemoglobin is preferably used.

In addition, as the light source 110, a laser or a light emitting diode may be used. Various lasers including a solid-state laser, a gas laser, a dye laser, and a semiconductor laser may be used as the laser. For example, as a laser used in this exemplary embodiment, an alexandrite laser, an yttrium-aluminum-garnet laser, or a titanium-sapphire laser may be used. Optical System 120

Light emitted from the light source 110 is guided to the living body 100 while being changed typically by optical components such as a lens and a mirror to have a desired light distribution shape. In addition, the light may also be propagated using a light waveguide such as an optical fiber or the like. Examples of the optical components include a mirror that reflects light, a lens that changes the shape of light by collecting or diverging light, a prism that disperses, refracts, and reflects light, an optical fiber in which light propagates, a diffusion plate that diffuses light, and the like. Anything may be used as such an optical component as long as an object is irradiated with light having a desired shape and emitted from the light source 110.

Note that, in the case where light emitted from the light source 110 is light having a desired shape, it is not necessary to use the optical system 120.

Acoustic Wave Detection Unit 130

The acoustic wave detection unit 130 includes a transducer and a housing. The transducer is a device capable of receiving an acoustic wave, and the housing covers the transducer.

The transducer receives an acoustic wave such as a photoacoustic wave or an ultrasonic echo and converts the acoustic wave into an electric signal, which is an analog signal. The transducer may be any of transducers using a piezoelectric phenomenon, optical resonance, a change in capacitance, or the like, as long as the transducer is capable of receiving an acoustic wave. In addition, the acoustic wave detection unit 130 preferably includes a plurality of transducers arranged in an array.

Processing Unit 140

The processing unit 140 includes the arithmetic section 141 and the memory section 142 as illustrated in Fig. 2.

The arithmetic section 141 is typically constituted by an arithmetic unit such as a central processing unit (CPU), a graphics processing unit (GPU), an analog-to-digital (A/D) converter, a field programmable gate array (FPGA), or an application-specific integrated circuit (ASIC). Note that the arithmetic section 141 may be constituted not only by a single arithmetic unit but also by a plurality of arithmetic units.

The memory section 142 is typically constituted by a storage medium such as a read-only memory (ROM) or a random-access memory (RAM). Note that the memory section 142 may be constituted not only by a single storage medium but also by a plurality of storage mediums.

In addition, the arithmetic section 141 may perform gain adjustment to obtain an image having a uniform contrast regardless of the depth inside a living body. In the gain adjustment, amplification gain is increased or decreased in accordance with a time period from when irradiation is performed with light to when an acoustic wave reaches an element of the acoustic wave detection unit 130.

In addition, the arithmetic section 141 may control a light emission timing of pulsed light emitted from the light source 110 and control, using pulsed light as a trigger, a timing of starting of operation of the acoustic wave detection unit 130.

In addition, in the case where a plurality of detection signals are obtained from the acoustic wave detection unit 130, the arithmetic section 141 is preferably configured to be able to simultaneously perform pipeline processing on a plurality of signals. As a result, a time period necessary to acquire object information may be shortened.

Note that pieces of processing to be performed by the processing unit 140 may be stored as programs to be executed by the arithmetic section 141, in the memory section 142.

In addition, the processing unit 140 and the acoustic wave detection unit 130 may be integrated into a single unit. Note that, here, part of processing may be performed by a processing unit included in the acoustic wave detection unit 130 and the remaining of the processing may be performed by a processing unit provided outside the acoustic wave detection unit 130. In this case, the processing unit included in the acoustic wave detection unit 130 and the processing unit provided outside the acoustic wave detection unit 130 may be collectively treated as the processing unit 140 according to this exemplary embodiment of the present invention.

Input Unit 150

The input unit 150 is configured to be able to receive an input from a user. Input information input by a user is input from the input unit 150 to the processing unit 140.

For example, a pointing device such as a mouse or a keyboard, a pen tablet type device, or the like may be used as the input unit 150. In addition, as the input unit 150, a button, a dial, or the like provided to a device included in the object information acquisition apparatus or a device outside the object information acquisition apparatus may also be used. In addition, in the case where the display unit 160 is a touch panel display, the display unit 160 may also function as the input unit 150.

Note that, the input unit 150 may also be provided separately from the object information acquisition apparatus according to this exemplary embodiment of the present invention. Display unit 160

The display unit 160 is a device that displays, as a distribution or numeric data, object information output from the processing unit 140.

A liquid crystal display or the like is typically used as the display unit 160; however, a display using another method such as a plasma display, an organic electroluminescence (EL) display, or a field emission display (FED) may also be used. In addition, by using a touch panel display as the display unit 160, the input unit 150 and the display unit 160 may be integrated into a single unit.

Note that the display unit 160 may also be provided separately from the object information acquisition apparatus according to this exemplary embodiment of the present invention. First Example

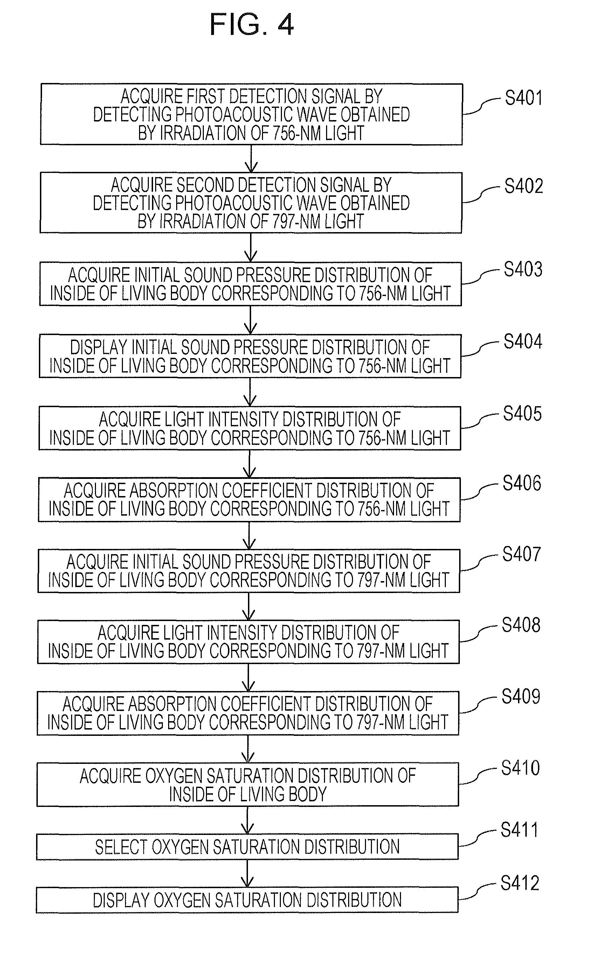

Next, an object information acquisition method according to a first example will be described with reference to Fig. 4. Note that the object information acquisition apparatus illustrated in Figs. 1 and 2 is used in the first example.

In the first example, an oxygen saturation distribution of the inside of the living body 100 is acquired using light of 756 nm, which is the first wavelength, and light of 797 nm, which is the second wavelength.

In the first example, an alexandrite laser capable of generating light of 756 nm (the first wavelength) and light of 797 nm (the second wavelength) is used as the light source 110.

In addition, in the first example, a substantial total amount of light from a light emission end of the optical system 120 is 70 mJ at two areas each of which having a size of 5 mm x 30 mm. The density of irradiation energy is, at the maximum, of the order of a maximum permissible exposure (MPE) multiplied by 0.8, the MPE being set for skin and described in International Electrotechnical Commission (IEC) 60285-1.

In addition, in the first example, a detector constituted by piezoelectric elements arranged in a linear array is used as the acoustic wave detection unit 130, the piezoelectric elements arranged in a linear array having a peak in receiving sensitivity for an acoustic wave of a frequency of 7 MHz.

S401: Process for Acquiring First Detection Signal by Detecting Photoacoustic Wave Obtained by Irradiation of 756-nm Light

In this process, first, the light source 110 emits light of 756 nm (the first wavelength). The living body 100 is irradiated with light of 756 nm as the pulsed light 121 via the optical system 120. Here, the arithmetic section 141 stores irradiation data of the pulsed light 121 of 756 nm in the memory section 142. The irradiation data is, for example, a light intensity distribution or an irradiation spot.

Next, the arithmetic section 141 causes the acoustic wave detection unit 130 to start reception of the photoacoustic wave 102, by taking emission of the pulsed light 121 as a trigger. Then, the acoustic wave detection unit 130 detects the photoacoustic wave 102 and outputs the first detection signal to the processing unit 140.

Next, processing such as amplification, A/D conversion, and the like is performed by the arithmetic section 141 on the first detection signal output from the acoustic wave detection unit 130. The resulting data is stored as first detection signal data in the memory section 142.

S402: Process for Acquiring Second Detection Signal by Detecting Photoacoustic Wave Obtained by Irradiation of 797-nm Light

In this process, first, the light source 110 emits light of 797 nm (the second wavelength). The living body 100 is irradiated with light of 797 nm as the pulsed light 121 via the optical system 120. Here, the arithmetic section 141 stores irradiation data of the pulsed light 121 of 797 nm in the memory section 142.

Next, the arithmetic section 141 causes the acoustic wave detection unit 130 to start reception of the photoacoustic wave 102, by taking emission of the pulsed light 121 as a trigger. Then, the acoustic wave detection unit 130 detects the photoacoustic wave 102 and outputs the second detection signal to the processing unit 140.

Then, processing such as amplification, A/D conversion, and the like is performed by the arithmetic section 141 on the second detection signal output from the acoustic wave detection unit 130. The resulting data is stored as second detection signal data in the memory section 142.

S403: Process for Acquiring Initial Sound Pressure Distribution of Inside of Living Body Corresponding to 756-nm Light

In this process, the arithmetic section 141 reads an image reconstruction algorithm stored in the memory section 142, and calculates an initial sound pressure distribution of the inside of the living body 100 by performing processing based on the read image reconstruction algorithm on the first detection signal data, the initial sound pressure distribution corresponding to light of 756 nm. Then, the arithmetic section 141 stores initial sound pressure distribution data of the inside of the living body 100 in the memory section 142, the initial sound pressure distribution data corresponding to light of 756 nm.

In the first example, the arithmetic section 141 performs, as processing based on the image reconstruction algorithm, universal back-projection (UBP) described in Non-Patent Document 1 (Minghua Xu and Lihong V. Wang, "Universal back-projection algorithm for photoacoustic computed tomography", PHYSICAL REVIEW E 71, 016706(2005)), the UBP being a type of back projection method performed in the time domain.

S404: Process for Displaying Initial Sound Pressure Distribution of Inside of Living Body Corresponding to 756-nm Light

In this process, the arithmetic section 141 reads the initial sound pressure distribution data stored in the memory section 142 and corresponding to light of 756 nm, and performs luminance value conversion processing on the initial sound pressure distribution data. Then, the arithmetic section 141 outputs, to the display unit 160, the resulting initial sound pressure distribution data corresponding to light of 756 nm, and causes the display unit 160 to display an initial sound pressure distribution corresponding to light of 756 nm.

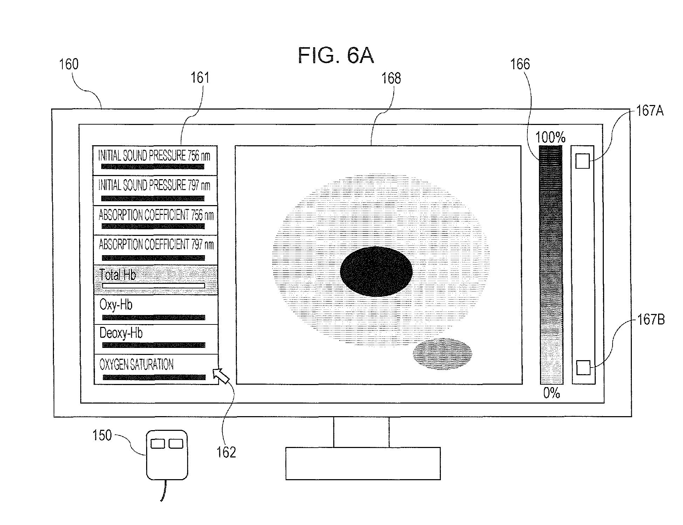

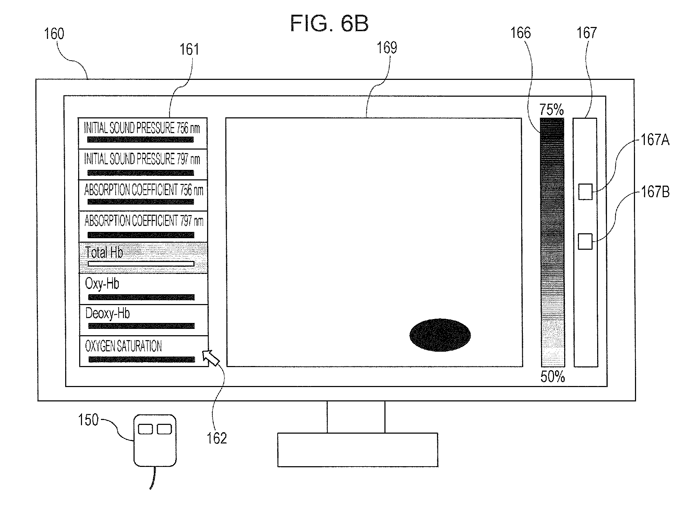

Fig. 6 illustrates the initial sound pressure distribution corresponding to light of 756 nm and displayed on the display unit 160 in this process. On the display unit 160, an initial sound pressure distribution 165 corresponding to light of 756 nm is displayed. The upper limit and lower limit of a dynamic range of the initial sound pressure distribution 165 may be adjusted by changing the position of an upper-limit setting slide bar 167A and that of a lower-limit setting slide bar 167B, respectively. The luminance value of the initial sound pressure distribution 165 is adjusted in accordance with adjustment of this dynamic range. In this manner, a user may check the initial sound pressure distribution 165 at a luminance arbitrarily set by the user.

In addition, for each type of object information, a progress bar representing calculation progress of the type of object information is displayed on the display unit 160. In this process, a progress bar 163 representing calculation progress of an initial sound pressure corresponding to light of 756 nm indicates that the calculation of the initial sound pressure corresponding to light of 756 nm has already been completed. In contrast, a progress bar 164 representing calculation progress of an initial sound pressure corresponding to light of 797 nm is partway filled with black color since the initial sound pressure corresponding to light of 797 nm is being calculated.

Note that, in the first example, the dynamic range of the initial sound pressure distribution 165 is initially set such that the initial sound pressure distribution 165 from 0 Pa to 65 Pa may be displayed. A color bar 166 corresponding to the dynamic range is displayed on the display unit 160.

In addition, in the first example, the initial sound pressure distribution 165 corresponding to light of 756 nm is initially set to be first displayed. Thus, the initial sound pressure distribution 165 corresponding to light of 756 nm is calculated and also displayed on the display unit 160.

Note that an initial sound pressure is information based on an absorption coefficient of the inside of a living body as expressed in Equation 1. Thus, as in the first example, an initial sound pressure distribution obtained using light of a wavelength (756 nm) at which hemoglobin absorbs a great amount of light is information effective for allowing a user to determine the shape of a blood vessel. That is, display of an initial sound pressure distribution allows a user to determine the shape of a blood vessel, thereby contributing to detection of the presence of a malignant tumor.

In the initial sound pressure distribution 165 according to the first example, an area 165A where there is a large amount of blood and an area 165B that surrounds the area 165A and where there is a small amount of blood may be determined. Furthermore, an area 165C where there is a small amount of blood may also be determined in the initial sound pressure distribution 165.

S405: Process for Acquiring Light Intensity Distribution of Inside of Living Body Corresponding to 756-nm Light

In this process, the arithmetic section 141 reads irradiation data stored in the memory section 142 and corresponding to light of 756 nm. The arithmetic section 141 calculates, using the irradiation data, a light intensity distribution of the inside of the living body 100, the light intensity distribution corresponding to light of 756 nm. Then, the arithmetic section 141 stores light intensity distribution data of the inside of the living body 100 in the memory section 142, the light intensity distribution data corresponding to light of 756 nm.

In the first example, the arithmetic section 141 calculates the light intensity distribution data of the inside of the living body 100, the light intensity distribution data corresponding to light of 756 nm, by performing analysis on the basis of an optical diffusion equation described in Non-Patent Document 2 (Yukio YAMADA et al., "Light-tissue interaction and optical imaging in biomedicine", Journal of Mechanical Engineering Laboratory, January, 1995, Vol. 49, No. 1, pp. 1-31).

Note that, in the first example of the present invention, a light intensity distribution may be acquired by performing analysis based on a Monte Carlo method or an optical transport equation other than by performing analysis based on an optical diffusion equation.

S406: Process for Acquiring Absorption Coefficient Distribution of Inside of Living Body Corresponding to 756-nm Light

In this process, using Equation 1, the arithmetic section 141 divides the initial sound pressure distribution data of the inside of the living body 100 by the light intensity distribution data of the inside of the living body 100, the initial sound pressure distribution data and the light intensity distribution data corresponding to light of 756 nm and being stored in the memory section 142. As a result, an absorption coefficient distribution of the inside of the living body 100 is calculated, the absorption coefficient distribution corresponding to light of 756 nm. Then, the arithmetic section 141 stores, in the memory section 142, absorption coefficient distribution data of the inside of the living body 100, the absorption coefficient distribution data corresponding to light of 756 nm.

S407: Process for Acquiring Initial Sound Pressure Distribution of Inside of Living Body Corresponding to 797-nm Light

In this process, the arithmetic section 141 reads the image reconstruction algorithm stored in the memory section 142. The arithmetic section 141 calculates an initial sound pressure distribution of the inside of the living body 100 by performing processing based on the read image reconstruction algorithm on the second detection signal data, the initial sound pressure distribution corresponding to light of 797 nm. Then, the arithmetic section 141 stores, in the memory section 142, initial sound pressure distribution data of the inside of the living body 100 the initial sound pressure distribution data corresponding to light of 797 nm.

In this process, too, the arithmetic section 141 performs the UBP described in Non-Patent Document 1 as processing based on the image reconstruction algorithm.

S408: Process for Acquiring Light Intensity Distribution of Inside of Living Body Corresponding to light of 797 nm

In this process, the arithmetic section 141 reads irradiation data stored in the memory section 142 and corresponding to light of 797 nm. The arithmetic section 141 calculates, using the irradiation data, a light intensity distribution of the inside of the living body 100, the light intensity distribution corresponding to light of 797 nm. Then, the arithmetic section 141 stores, in the memory section 142, light intensity distribution data of the inside of the living body 100, the light intensity distribution data corresponding to light of 797 nm.

In the first example, the arithmetic section 141 calculates the light intensity distribution data of the inside of the living body 100 by performing analysis on the basis of an optical diffusion equation described in Non-Patent Document 2, the light intensity distribution data corresponding to light of 797 nm.

S409: Process for Acquiring Absorption Coefficient Distribution of Inside of Living Body Corresponding to 797-nm Light

In this process, using Equation 1, the arithmetic section 141 divides the initial sound pressure distribution data of the inside of the living body 100 by the light intensity distribution data of the inside of the living body 100, the initial sound pressure distribution data and the light intensity distribution data corresponding to light of 797 nm and being stored in the memory section 142. As a result, an absorption coefficient distribution of the inside of the living body 100 is calculated, the absorption coefficient distribution corresponding to light of 797 nm. Then, the arithmetic section 141 stores, in the memory section 142, absorption coefficient distribution data of the inside of the living body 100, the absorption coefficient distribution data corresponding to light of 797 nm.

S410: Process for Acquiring Oxygen Saturation Distribution of Inside of Living Body

In this process, the arithmetic section 141 calculates an oxygen saturation distribution of the inside of the living body 100, using the absorption coefficient distributions of the inside of the living body 100, the absorption coefficient distributions corresponding to light of 756 nm and 797 nm and being stored in the memory section 142.

Note that the arithmetic section 141 first calculates, using Equation 3, the density of oxyhemoglobin and the density of deoxyhemoglobin in the first example.

Then, using Equation 4, the arithmetic section 141 calculates oxygen saturation, using the density of oxyhemoglobin and the density of deoxyhemoglobin.

Then, the arithmetic section 141 stores calculated oxyhemoglobin density distribution data, calculated deoxyhemoglobin density distribution data, and calculated oxygen saturation distribution data in the memory section 142.

S411: Selection of Oxygen Saturation Distribution

In this process, as illustrated in Fig. 6A, a user operates an arrow 162 displayed on the display unit 160 with a mouse serving as the input unit 150, and selects an item corresponding to oxygen saturation from a menu 161 that is displayed on the display unit 160 illustrated in Fig. 6A and shows certain types of object information. Specifically, a user operates the arrow 162 with a mouse and then clicks a button equipped with the mouse when the arrow 162 overlaps an item representing oxygen saturation. Note that in the first example, the menu 161 for an object includes items representing initial sound pressure distributions and absorption coefficient distributions corresponding to wavelengths of 756 nm and 797 nm. The menu 161 also includes items representing an oxyhemoglobin density distribution, a deoxyhemoglobin density distribution, and an oxygen saturation distribution. That is, the input unit 150 is configured to be able to make a selection from among initial sound pressure distribution data and absorption coefficient distribution data corresponding to the wavelengths of 756 nm and 797 nm, oxyhemoglobin density distribution data, deoxyhemoglobin density distribution data, and oxygen saturation distribution data.

Note that, since a total hemoglobin density distribution is not calculated in the first example, an item corresponding to the total hemoglobin density distribution is displayed with a gray background and a user is not allowed to select this item. In contrast, since calculation has already been performed for items regarding object information other than the total hemoglobin density distribution in the first example, these items are displayed with a white background and a user may select these items.

S412: Display Oxygen Saturation Distribution

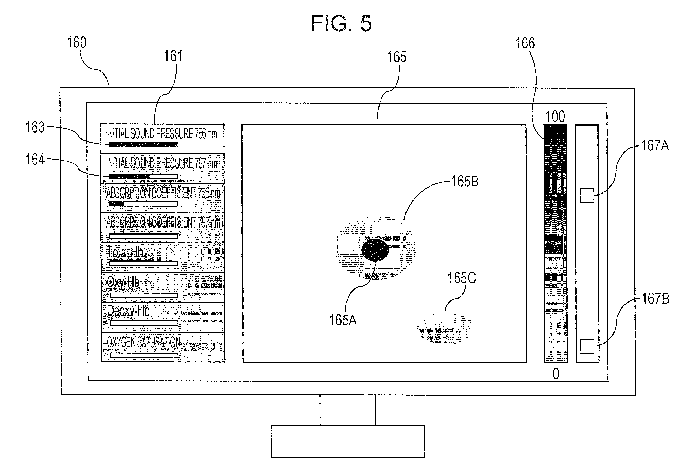

Next, the arithmetic section 141 reads, from the memory section 142, the oxygen saturation distribution data from among the initial sound pressure distribution data and absorption coefficient distribution data corresponding to the wavelengths of 756 nm and 797 nm, the oxyhemoglobin density distribution data, the deoxyhemoglobin density distribution data, and the oxygen saturation distribution data stored in the memory section 142. Subsequently, the arithmetic section 141 performs luminance value conversion processing on the oxygen saturation distribution data, and outputs the resulting oxygen saturation distribution data to the display unit 160. Subsequently, the display unit 160 displays an oxygen saturation distribution 168 of the inside of the living body 100 as illustrated in Fig. 6A such that the initial sound pressure distribution 165 illustrated in Fig. 5 is switched to the oxygen saturation distribution 168.

In addition, the arithmetic section 141 assigns luminance values each of which corresponds to a corresponding one of 0% to 100% to the oxygen saturation distribution data on the basis of a gray scale displayed in the color bar 166, and causes the display unit 160 to display the oxygen saturation distribution 168.

In addition, in the first example, as illustrated in Fig. 6B, a user operates the upper-limit setting slide bar 167A and changes the upper limit of oxygen saturation to be displayed from 100% to 75%. In addition, a user operates the lower-limit setting slide bar 167B, which is displayed, and changes the lower limit of oxygen saturation from 0% to 50%. Then, the arithmetic section 141 assigns luminance values each of which corresponds to a corresponding one of 50% to 75% to the oxygen saturation distribution data on the basis of a gray scale displayed in the color bar 166, and causes the display unit 160 to display an oxygen saturation distribution 169.

As in the first example, a user may know an oxygen saturation distribution by causing a display unit to display the oxygen saturation distribution, thereby helping making of a diagnosis of the presence of a malignant tumor.

Furthermore, in the first example, a user first determines the shapes of blood vessels from a displayed initial sound pressure distribution. Thereafter, display is performed such that the displayed initial sound pressure distribution is switched to an oxygen saturation distribution, and consequently the user may make a diagnosis by collectively taking pieces of information having different pathological values into account. Other Embodiments

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment(s) of the present invention, and by a method performed by the computer of the system or apparatus by, for example, reading out and executing the computer executable instructions from the storage medium to perform the functions of one or more of the above-described embodiment(s). The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

An object information acquisition apparatus disclosed in the present specification includes a light source (110), acoustic wave detection means (130), and processing means (140). The light source (110) is configured to emit light. The acoustic wave detection means (130) is configured to detect a photoacoustic wave generated by irradiation of an object with the light and to output a detection signal. The processing means (140) is configured to be able to acquire two or more types of object information on the basis of the detection signal and configured to cause display means (160) to display at least one type of object information selected by a user from among the two or more types of object information.

**EP 2 749 209 A1**

**Claims**

1. An object information acquisition apparatus comprising:

   a light source (110) configured to emit light;
   acoustic wave detection means (130) configured to detect a photoacoustic wave generated by irradiation of an object with the light and to output a detection signal; and
   processing means (140) configured to be able to acquire two or more types of object information on the basis of the detection signal and configured to cause display means (160) to display at least one type of object information selected by a user from among the two or more types of object information.

2. The object information acquisition apparatus according to Claim 1, wherein
   the light source (110) emits light of a plurality of wavelengths,
   the acoustic wave detection means (130) detects photoacoustic waves generated by irradiation of the object with light of the plurality of wavelengths and outputs detection signals corresponding to light of the plurality of wavelengths, each of the photoacoustic waves corresponding to light of a corresponding one of the plurality of wavelengths and each of the detection signals corresponding to light of a corresponding one of the plurality of wavelengths, and
   the processing means (140) is configured to be able to acquire the two or more types of object information, which include morphological information and spectral information, on the basis of the detection signals corresponding to light of the plurality of wavelengths.

3. The object information acquisition apparatus according to Claim 2, wherein the processing means (140) acquires morphological information on the basis of a detection signal corresponding to light of a certain wavelength among light of the plurality of wavelengths.

4. The object information acquisition apparatus according to Claim 2 or 3, wherein the morphological information includes at least one of an initial sound pressure distribution and an absorption coefficient distribution.

5. The object information acquisition apparatus according to any one of Claims 2 to 4, wherein the processing means (140) acquires the spectral information on the basis of detection signals corresponding to light of at least two wavelengths among light of the plurality of wavelengths.

6. The object information acquisition apparatus according to Claim 2 or 5, wherein the spectral information includes any one of an oxygen saturation distribution, an oxyhemoglobin density distribution, and a deoxyhemoglobin density distribution.

7. The object information acquisition apparatus according to any one of Claims 1 to 6, wherein, when the at least one type of object information is selected by a user, the processing means (140) causes the display means (160) to display the at least one type of object information, which is stored in storage means (142).

8. The object information acquisition apparatus according to any one of Claims 1 to 6, wherein, when the at least one type of object information is selected by a user, the processing means (140) acquires the at least one type of object information and causes the display means (160) to display the at least one type of object information.

9. The object information acquisition apparatus according to any one of Claims 1 to 8, further comprising the display means (160).

10. The object information acquisition apparatus according to any one of Claims 1 to 9, further comprising input means (150) configured to allow a user to select the at least one type of object information from among the two or more types of object information.

11. The object information acquisition apparatus according to Claim 1, wherein the processing means (140) is configured to be able to acquire three or more types of object information on the basis of the detection signal, and causes the display means (160) to display at least two types of object information selected by a user from among the three or more types of object information.

12. A display method, comprising:

receiving (S303) input information on at least one type of object information among two or more types of object information;

acquiring (S302) the at least one type of object information on the basis of a detection signal of a photoacoustic wave generated by irradiation of an object with light; and

displaying (S304) the at least one type of object information.

13. The display method according to Claim 12, wherein
the acquiring (S302) is performed after the receiving (S303), and
the displaying (S304) is performed after the acquiring (S302).

14. The display method according to Claim 12, wherein
the receiving (S303) is performed after the acquiring (S302), and
the displaying (S304) is performed after the receiving (S303).

15. A program causing a computer to execute the display method according to any one of Claims 12 to 14.

# FIG. 1

# FIG. 2

# FIG. 3

ACQUIRE DETECTION SIGNAL BY DETECTING PHOTOACOUSTIC WAVE — S301

ACQUIRE TWO OR MORE TYPES OF OBJECT INFORMATION ON THE BASIS OF DETECTION SIGNAL — S302

SELECT DESIRED OBJECT INFORMATION FROM AMONG TWO OR MORE TYPES OF OBJECT INFORMATION — S303

DISPLAY DESIRED OBJECT INFORMATION — S304

# FIG. 4

ACQUIRE FIRST DETECTION SIGNAL BY DETECTING PHOTOACOUSTIC WAVE OBTAINED BY IRRADIATION OF 756-NM LIGHT — S401

ACQUIRE SECOND DETECTION SIGNAL BY DETECTING PHOTOACOUSTIC WAVE OBTAINED BY IRRADIATION OF 797-NM LIGHT — S402

ACQUIRE INITIAL SOUND PRESSURE DISTRIBUTION OF INSIDE OF LIVING BODY CORRESPONDING TO 756-NM LIGHT — S403

DISPLAY INITIAL SOUND PRESSURE DISTRIBUTION OF INSIDE OF LIVING BODY CORRESPONDING TO 756-NM LIGHT — S404

ACQUIRE LIGHT INTENSITY DISTRIBUTION OF INSIDE OF LIVING BODY CORRESPONDING TO 756-NM LIGHT — S405

ACQUIRE ABSORPTION COEFFICIENT DISTRIBUTION OF INSIDE OF LIVING BODY CORRESPONDING TO 756-NM LIGHT — S406

ACQUIRE INITIAL SOUND PRESSURE DISTRIBUTION OF INSIDE OF LIVING BODY CORRESPONDING TO 797-NM LIGHT — S407

ACQUIRE LIGHT INTENSITY DISTRIBUTION OF INSIDE OF LIVING BODY CORRESPONDING TO 797-NM LIGHT — S408

ACQUIRE ABSORPTION COEFFICIENT DISTRIBUTION OF INSIDE OF LIVING BODY CORRESPONDING TO 797-NM LIGHT — S409

ACQUIRE OXYGEN SATURATION DISTRIBUTION OF INSIDE OF LIVING BODY — S410

SELECT OXYGEN SATURATION DISTRIBUTION — S411

DISPLAY OXYGEN SATURATION DISTRIBUTION — S412

FIG. 5

# FIG. 6A

## FIG. 6B

INITIAL SOUND PRESSURE 756 nm

INITIAL SOUND PRESSURE 797 nm

ABSORPTION COEFFICIENT 756 nm

ABSORPTION COEFFICIENT 797 nm

Total Hb

Oxy-Hb

Deoxy-Hb

OXYGEN SATURATION

75%

50%

Europäisches
Patentamt

European
Patent Office

Office européen
des brevets

## EUROPEAN SEARCH REPORT

Application Number

EP 13 19 7073

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/066023 A1 (KANAYAMA SHOICHI [JP] ET AL) 17 March 2011 (2011-03-17) <br> * paragraph [0101] - paragraph [0104] * <br> * paragraph [0054] - paragraph [0058] * <br> * paragraph [0077] - paragraph [0080] * <br> * paragraph [0090]; claims 1-27; figures 1-2 * | 1-15 | INV. <br> A61B5/00 <br> G01N29/24 <br> G01N21/17 |
| X | US 2012/203093 A1 (IMRAN MIR [US] ET AL) 9 August 2012 (2012-08-09) <br> * claims 1-50; figures 1-5 * | 1-6,12, 15 | |
| X | WO 2012/086842 A1 (CANON KK [JP]; OISHI TAKUJI [JP]) 28 June 2012 (2012-06-28) <br> * paragraph [0004] - paragraph [0006] * <br> * paragraph [0048] * <br> * paragraph [0040] - paragraph [0042] * <br> * abstract; claims 1-13; figures 1-16 * | 1-6 | |
| E | WO 2014/013867 A1 (CANON KK [JP]) 23 January 2014 (2014-01-23) <br> * paragraph [0033]; claims 1-12; figures 1-10 * <br> * paragraph [0130] - paragraph [0139] * <br> * paragraph [0112] * | 1,12,15 | TECHNICAL FIELDS SEARCHED (IPC) <br> A61B <br> G01N |
| X | US 2011/190617 A1 (CHEN JINGKUANG [US]) 4 August 2011 (2011-08-04) <br> * paragraph [0012] * <br> * paragraph [0013] * <br> * paragraph [0014] * <br> * paragraph [0017] * <br> * paragraph [0079]; figure 1 * <br> * paragraph [0023] - paragraph [0025] * | 1,12,15 | |
| X | US 2012/220844 A1 (BAKER JR CLARK R [US]) 30 August 2012 (2012-08-30) <br> * paragraphs [0035], [0039]; claims 1-19; figures 1-3 * | 1-6,12, 15 | |

The present search report has been drawn up for all claims

| Place of search | Date of completion of the search | Examiner |
|---|---|---|
| Munich | 25 March 2014 | Apostol, Simona |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
.......................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 13 19 7073

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2014

| Patent document cited in search report | | Publication date | Patent family member(s) | | Publication date |
|---|---|---|---|---|---|
| US 2011066023 | A1 | 17-03-2011 | CA | 2462378 A1 | 06-08-2005 |
| | | | CN | 1650794 A | 10-08-2005 |
| | | | EP | 1561424 A1 | 10-08-2005 |
| | | | JP | 4643153 B2 | 02-03-2011 |
| | | | JP | 2005218684 A | 18-08-2005 |
| | | | KR | 20050079610 A | 10-08-2005 |
| | | | US | 2005187471 A1 | 25-08-2005 |
| | | | US | 2011066023 A1 | 17-03-2011 |
| US 2012203093 | A1 | 09-08-2012 | US | 2012203093 A1 | 09-08-2012 |
| | | | WO | 2012109394 A2 | 16-08-2012 |
| WO 2012086842 | A1 | 28-06-2012 | CN | 103260502 A | 21-08-2013 |
| | | | EP | 2654550 A1 | 30-10-2013 |
| | | | JP | 2012143547 A | 02-08-2012 |
| | | | US | 2013261427 A1 | 03-10-2013 |
| | | | WO | 2012086842 A1 | 28-06-2012 |
| WO 2014013867 | A1 | 23-01-2014 | JP | 2014018369 A | 03-02-2014 |
| | | | WO | 2014013867 A1 | 23-01-2014 |
| US 2011190617 | A1 | 04-08-2011 | EP | 2281188 A2 | 09-02-2011 |
| | | | US | 2011190617 A1 | 04-08-2011 |
| | | | WO | 2009158146 A2 | 30-12-2009 |
| US 2012220844 | A1 | 30-08-2012 | CA | 2827981 A1 | 29-11-2012 |
| | | | EP | 2680753 A1 | 08-01-2014 |
| | | | US | 2012220844 A1 | 30-08-2012 |
| | | | WO | 2012161841 A1 | 29-11-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010088627 A

### Non-patent literature cited in the description

- **MINGHUA XU ; LIHONG V. WANG.** Universal back-projection algorithm for photoacoustic computed tomography. *PHYSICAL REVIEW E,* 2005, vol. 71, 016706

- **YUKIO YAMADA et al.** Light-tissue interaction and optical imaging in biomedicine. *Journal of Mechanical Engineering Laboratory,* January 1995, vol. 49 (1), 1-31